(19) Europäisches Patentamt
European Patent Office
Office européen des brevets

(11) Publication number: 0 156 772
A2

(12) **EUROPEAN PATENT APPLICATION**

(21) Application number: 85810123.1

(22) Date of filing: 22.03.85

(51) Int. Cl.$^4$: A 61 K 37/30
//C07K7/10

(30) Priority: 27.03.84 GB 8407907

(43) Date of publication of application:
02.10.85 Bulletin 85/40

(84) Designated Contracting States:
BE CH DE FR GB IT LI LU NL SE

(71) Applicant: SANDOZ AG
Lichtstrasse 35
CH-4002 Basel(CH)

(84) Designated Contracting States:
BE CH FR GB IT LI LU NL SE

(71) Applicant: SANDOZ-PATENT-GMBH
Humboldtstrasse 3
D-7850 Lörrach(DE)

(84) Designated Contracting States:
DE

(72) Inventor: Azria, Moise
Bundesplatz 6
CH-4054 Basel(CH)

(72) Inventor: MacIntyre, Iain
Great Broadhurst Farm Broad Oak
Heathfield East Sussex, TN21 8UX(GB)

(54) **Novel pharmaceutical use for CGRPs.**

(57) CGRPs, for example human and rat CGRP, and related peptides are effective as regulators of body Ca balance and are useful e,g, for the treatment of Paget's disease, osteoporosis and other related conditions. For this purpose they may be administered alone or in conjunction with a calcitonin, for example human or salmon calcitonin.

EP 0 156 772 A2

Case 100-6306

## NOVEL PHARMACEUTICAL USE FOR CGRPs

The present invention relates to a new use, in particular a new pharmaceutical use, for peptides comprising a CGRP peptide sequence, and analogues thereof.

CGRP (Calcitonin Gene Related Peptide) is the term applied to a class of peptide produced by variant expression of calcitonin genes, e.g. in neural tissue or peripheral thyroid tissue.

The calcitonins themselves constitute a class of peptide hormone found to occur in a wide variety of chordate species. In mammals calcitonins are secreted from the thyroid gland and in non-mammalian species from the ultimobranchial gland. Though of closely related structure, precise calcitonin peptide sequence has been found to vary to a greater or lesser degree from species to species and is generally species specific.

Calcitonins inhibit bone-resorption with consequential induction of hypocalcaemia, and various proposals have been made for the therapeutic application of both calcitonins of naturally occurring structure and artificial analogues thereof. Salmon calcitonin, human calcitonin, porcine calcitonin, bovine calcitonin and the eel calcitonin analogue "Elcatonin" are now commercially available and, salmon calcitonin in particular has found extensive and valuable clinical application.

In mammals calcitonin is produced by the thyroidal c-cells. Recently the gene system for calcitonins has been the subject of intensive study. This has led to the elucidation of the human calcitonin structural gene (published European patent application EP-AI-0070675) and to the isolation of calcitonin precursor peptides and further peptide entities, such as katacalcin, derived from the flanking sequences of the human calcitonin precursor peptide (published European patent application EP-AI-0070186).

Still more recently a yet further class of peptide entity has been identified: the so-called calcitonin gene related peptides or CGRPs - see e.g. Amara et al., Nature 298, 240 - 244 (1982); Rosenfeld et al., Nature 304, 129 - 135 (1983); Fischer et al., Nature 305, 534 - 536 (1983) concerning rat CGRP; and Morris et al., Nature 308, 746 - 748 (1984); MacIntyre et al., Neuropeptides 4, 425 - 484 (1984); Brain et al., Nature 313, 54 - 56 (1985); and MacIntyre et al., International (PCT) patent application no. 87/00307.

Structurally CGRPs represent an entirely new peptide class, with peptide sequences entirely distinct from those of the calcitonins. CGRPs have been found to be physiologically active and to exert a potent effect on mediator release, including release of neuro-transmitters. Utility has been proposed for e.g. human CGRP as a cardiovascular agent, in particular for the treatment of peripheral vascular disease (e.g. for the prophylaxis or treatment of disturbances in peripheral circulation, e.g. in limbs, including intermittant claudication) as well as for the treatment of coronary vessel disease (e.g. for the prophylaxis or treatment of coronary insufficiency). A further area of application indicated is in patient testing, e.g. in determining tone of the coronary arteries, e.g. in relation to application of other drug therapy or coronary surgery, e.g. coronary by-pass therapy.

(See in particular International (PCT) patent application no. 87/00307 referred to above).

In accordance with the present invention it has now surprisingly been found that CGRPs, also exhibit valuable $Ca^{2+}$ regulating activity. Yet more suprisingly it has been found that, quite unlike the calcitonins they exhibit both hypo- and hyper-calcaemic effects, e.g. as hereinafter described. This entirely unexpected opposition of activities renders the CGRPs especially suited for application as regulators of body $Ca^{2+}$ metabolism, e.g. in the treatment of disease associated with or having an aetiology comprising body $Ca^{2+}$ imbalance, in particular in the treatment of hypercalcaemia (including emergency treatment of hypercalcaemic crisis as well as prolonged treatment in chronic hypercalcaemic states), Paget's disease, and, especially, osteoporosis, including post-menopausal osteoporosis, immobilization osteoporosis, for example subsequent to fracture, rapid osteoporosis due to nerve paralysis and steroid induced osteoporosis, as well as bone pain associated with osteoporosis or osteolysis, and pain consequential to metastatic bone deposits.

The present invention accordingly provides:

i) A method for the regulation of calcium, e.g. for the treatment of disease associated with or having an aetiology comprising body calcium imbalance, in particular for the treatment of specific conditions as set forth above, in a subject in need of such treatment, which method comprises administering to said subject an effective amount of a peptide comprising the peptide sequence of a CGRP or of an analogue thereof;

ii) The use of a peptide comprising the peptide sequence of a CGRP or of an analogue thereof, for the regulation of calcium, e.g. for the treatment of disease associated with or having an aetiology comprising body calcium imbalance, in particular for the treatment of specific conditions as set forth above; as well as:

iii) The use of a peptide comprising the peptide sequence of a CGRP or of an analogue thereof, for the manufacture of a composition for use in the treatment of disease associated with or having an aetiology comprising body calcium imbalance, in particular for the treatment of specific conditions as set forth above;

By the term "peptide comprising the peptide sequence of a CGRP" as used herein is to be understood not only the CGRPs themselves but also precursors thereof, for example, the naturally occurring CGRP precursor peptides.

By "analogues" of CGRPs are meant peptides derived from the structure of a CGRP, e.g. obtained by any of the means commonly employed in the art of peptide chemistry for the derivatisation or modification of naturally occurring peptide entities, for example by omission of one or more amino acid residues within the natural peptide sequence, or by replacement of one or more such residues by an alternative amino acid residue, by inversion of one or more peptide units with the natural peptide sequence, or by derivatisation of the C- or N-terminal.

Preferred peptides for use in accordance with the present invention are rat CGRP and, in particular, human CGRP having the formula:

H-A-Cys-B—Thr-Ala-Thr-Cys-Val-Thr-His-Arg

Leu-Ala-Gly-Leu-Leu-Ser-Arg-Ser-Gly-Gly-Val

Val-Lys—C—Asn-Phe-Val-Pro-Thr-Asn-Val-Gly

Ser—D—Ala-Phe-$NH_2$.

wherein either:

A is -Ser-, B is -Asn-, C is -Asp- and D is -Glu- (RAT-CGRP); or
A is -Ala-, B is -Asp-, C is -Asn- and D is -Lys- (HUMAN CGRP).

The peptides for use in accordance with the present invention can be prepared in accordance with conventional techniques for peptide synthesis known in the art, including solid-phase synthesis, as well as by genetic engineering, e.g. recombinant DNA, techniques.

For use in accordance with the present invention the subject peptides may be employed in free or in pharmaceutically acceptable salt or complex form, for example in the form of their pharmaceutically acceptable acid addition salts (e.g. hydrochlorides, sulfonates, etc.) or metal complexes, such salt and complex forms being well known in the art. Such salt and complex forms generally have the same level of activity and tolerability as the free peptides. All references to the subject peptides herein are to be understood as including both the free peptides as well as their pharmaceutically acceptable salts and complexes as aforesaid.

Furthermore the subject peptides can, in accordance with the method of the invention, also be employed alone, or in conjunction with other appropriate pharmaceutical agents, in particular agents capable of regulating calcium metabolism, and especially in conjunction with calcitonins, e.g. as hereinafter described.

Utility of the subject peptides in accordance with the present invention may be demonstrated in animal test models as well as in clinical trials, e.g. performed as follows:

I. <u>CONTINUOUS ON-LINE MEASUREMENT OF FREE CALCIUM CONCENTRATIONS IN THE EXTRA-CORPOREAL BLOOD CIRCULATION OF YOUNG RABBITS:</u>

The general scheme for this test is represented in figure 1 attached:

A flow-through electrode system with two $Ca^{2+}$-selective liquid membrane electrodes is inserted in the arterio-venous extracorporeal blood circulatory system of the test animal this being established by means of indwelling catheters connecting the femoral artery with femoral vein.

The blood is pumped from the artery to the vein at a flow rate of 18 ml/h through the system containing $Ca^{2+}$-selective electrodes. A second peristaltic pump aspirates the reference solution of electrolytes, which is transported at a flow rate of 0.9 ml/h, passing the compartment of the reference electrode and mixed with the circulating blood at the site of the common electrode. The mixed fluids are then returned into the ciculatory system of the animal.

The $Ca^{2+}$-selective electrode allows the potentiometric measurement of the ionized calcium quantitatively. The potential difference between the reference electrode and the $Ca^{2+}$-selective electrode is measured differentially relative to the common electrode. The analogue signal of the difference amplifier is processed by a digital voltmeter. Results are obtained in mV and are continously recorded as a function of time. The voltage difference is proportional to the $Ca^{2+}$ concentration.

Following administration of CGRPs i.v. to the test animal, a potent influence on $Ca^{2+}$ concentration over extended periods of time and up to at least 5 hours is observed at dosages of from about 0.1 to about 25 µg/kg. More importantly results observed indicate that CGRPs exert both a hypo- and hyper-calcaemic effect which is both dose- and time-dependent. Thus administration of rat CGRP at dosages of 5 µg/kg or less induces a primarily hypocalcaemic response, while administration at dosages in excess of 5 µg/kg induces a primarily hyper-calcaemic response preceeded by a transient hypo-calcaemic response at the lower end of the dosage range. Specific results obtained following 1x administration of rat CGRP at varying dosages and illustrating activity profile are shown in the accompanying Figs. 2 through 5.

In all cases administration of test substance occurs at time ∅.

Fig.2 .: This is a plot for observed change in calcium level [Δ % calcaemia] with time, following i.v. administration of rat CGRP at dosages of from 0.156 μg/kg to 5.0 μg/kg. In all cases a decrease (hypocalcaemia) is observed with a duration of from 1 to at least 3 hours. Evaluation of AUCS (areas under curve) obtained indicate that these are statistically linear.

Figs3 to4 : Plots for observed change in calcium level following i.v. administration of rat CGRP at dosages of 2.5 and 5.0 μg/kg to groups of four test animals compared with results obtained for untreated controls. In both cases hypocalcaemia is again observed with a duration of from 3 to 6 hours following administration of rat CGRP.

Fig 5: A plot for observed change in calcium level following administration of rat CGRP at a dosage of 10 μg/kg i.v. compared with untreated control. At this dosage hypocalcaemic response analogous to that observed at lower dosages (Figs 1 to 3), though strongly marked, is transitory only and is followed by a reverse hypercalcaemic response which continues very strongly for a period of up to at least 5 hours following initial administration.

With further dosage increase hypercalcaemic response becomes increasingly dominant.

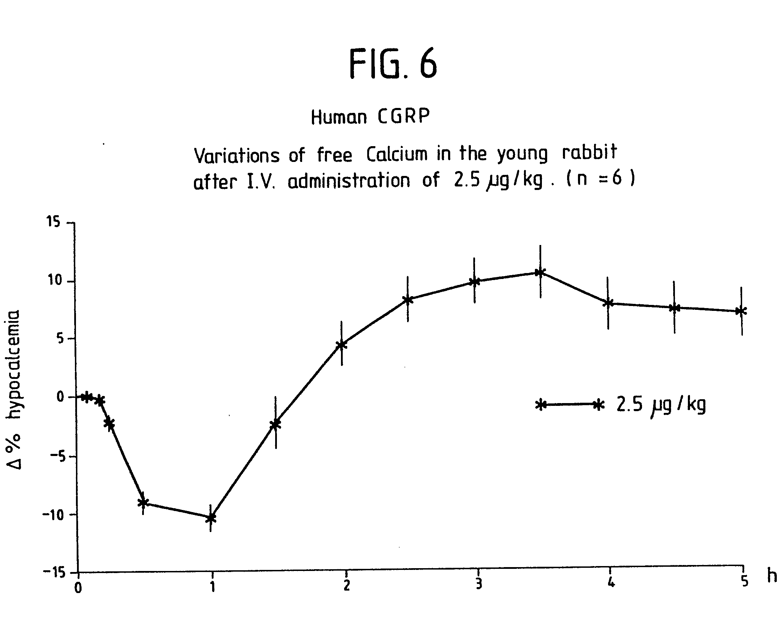

Directly analogous results may be obtained employing other CGRPs and peptides incorporating a CGRP peptide sequence, in particular employing human CGRP. Thus accompanying Fig. 6 shows plots for observed change in calcium level following i.v. administration of human CGRP at a dosage of 2.5 µg/kg to groups of 6 animals in the same test model as described above. As will be seen the course of response parallels that recorded for rat CGRP in Fig. 5.

These results indicate that the subject peptides exert an influence not only on the uptake of calcium into body tissue, but also on liberation of free calcium, enabling further subsequent up-take.

## II. CLINICAL TRIAL A:

The trial is carried out employing volunteer subjects (male and female) exhibiting active Paget's disease. Human CGRP is employed as trial substance and is tested by administration in accordance with the following regimens.

### 1. Acute (single dosage) administration:

1.1) i.v. injection of dosages of from 0.1 to 20 µg (e.g. 1x 0.1, 0.2, 0.5, 1.0, 5.0, 10, or 20 µg);

1.2) i.v. infusion over 6 hours of dosages of 10 to 100 µg (e.g. 1x 10, 15, 20, 25, 50 or 100 µg);

1.3) s.c. injection of dosages of from 10 to 200 µg (e.g. 1x 10, 20, 50, 100, 150 and 200 µg);

1.4) intranasal administration of dosages of from 10 to 500 µg (e.g. 1x 10, 50, 100, 150, 250 and 500 µg);

2. Semi-chronic (single dosage daily for 5 to 7 days) administration:

2.1) s.c. injection of dosages of from 10 - 200 µg (e.g. 10, 25, 50, 100, 150 and 200 µg 1x daily).

The following parameters are measured prior and post-administration: circulating calcium serum levels: alkaline phosphatase: hydroxyproline: bone specific protein levels.

On administration of human CGRP in accordance at the above indicated dosages, significant reduction of circulating serum calcium levels is observed. Assay for alkaline phosphatase and hydroxyproline levels show a concomitant reduction and assay for bone specific proteins shows a marked reduction in particular of those proteins reflecting bone metabolic turn over.

III. CLINICAL TRIAL B:

The methodology of clinical trial A is repeated, with administration of human CGRP in accordance with regimen 1.3. In this trial however administration is accompanied by administration of salmon or human calcitonin at dosages of from 5 to 100 I.U. (or M.R.C), [e.g. at dosages of 5, 10, 25, 50 or 100 I.U. (or M.R.C.)] administered by injection either s.c. or i.m.

Influence on measured serum parameters is observed in this trial as in trial A, the effect in relation to reduction of circulating serum calcium levels is in particular superior to that observed on administration of salmon calcitonin alone at the indicated dosages.

The subject peptides are accordingly useful for the treatment of disease associated with or having an aetiology comprising body $Ca^{2+}$ imbalance, in particular for the treatment of specific conditions hereinbefore set forth and especially for the treatment of osteoporosis.

For this use the dosage will of course vary depending on the particular peptide employed, the mode of administration, the particular condition to be treated, the duration of treatment envisioned and the effect desired. However, in general, satisfactory results are obtained on administration of, for example, human or rat CGRP, at a daily dosage of from about 0.001 to about 15.0, for example from about 0.05 to about 15.0 μg/kg, e.g. from about 0.07 to about 7.0 μg/kg, in particular from about 0.15 to about 3.0 μg/kg animal body weight. For clinical use the total daily dosage is suitably in the range of from about 0.1 to about 500 μg, for example from about 5.0 to about 500 μg, preferably from about 10 μg to about 200 μg e.g. for administration i.v., s.c., i.m. or nasally, whereby higher dosages, e.g. of up to about 1,000 μg may be employed, for example where intensive short term treatment or, in particular, nasal administration, is contemplated.

As indicated above dosages will depend inter al. on the particular condition to be treated. Thus for the treatment of osteoporosis lower dosages (e.g. at the lower end of the ranges indicated above) will generally be appropriate as compared with dosages required e.g. for the treatment of Paget's disease or pain consequential to metastatic bone deposits.

Administration may be effected by any of the routes commonly employed in the art in relation to administration of e.g. calcitonins, e.g. by injection or infusion i.v., by injection s.c. or i.m. or nasally, e.g. in the form of nasal drops or nasal spray. In general however i.v. administration will be less preferred. Administration may be efected e.g. 1x daily, in divided doses 2x to 4x daily or in sustained release form. Unit dosage forms for administration thus suitably comprise from about about 0.025 to about 250 μg, for example from 1.25 to about 250 μg, preferably from about 2.5 to about 100 μg for multiple daily dosaging, or up to about 500 μg, preferably about 200 μg for single daily dosaging, with higher unit dosages, e.g. of up to about 500 μg being possible, for example where intensive short term treatment/nasal administration is contemplated.

In accordance with the present invention the subject peptides, e.g. human and rat CGRP, may be administered alone or conjointly or concomitantly with other appropriate pharmaceutical agents, in particular with calcitonins, e.g. salmon, human, porcine or bovine calcitonin or the eel calcitonin analogue Elcatonin. Where conjoint or concomitant therapy with a calcitonin is required, daily dosages for the calcitonin will be of similar order, or slightly less than those employed for conventional calcitonin therapy, e.g. from about 5 to about 200 I.U. (or M.R.C.) per day, for example from about 5 to about 50, suitably from about 5 to about 25 I.U. (or M.R.C.) per day for the treatment of osteoporosis, or from about 25 to about 200, suitably from about 50 to about 100 I.U. (or M.R.C.) per day for the treatment of Paget's disease or pain consequential to metastatic bone deposits, administered 1x or 2-4x daily. Preferred calcitonins for conjoint or concomitant use with the peptides of the invention are salmon and human calcitonin.

Where administration with a calcitonin is to be practiced, the calcitonin may be administered either separately or together with the subject peptides, and if desired the subject peptides may be put up in combination with a calcitonin in the same galenical formulation. Where administration is to be effected 2 to 4x daily suitable amounts of calcitonin, e.g. salmon calcitonin, for inclusion in such combined dosage forms will be from about 1.25 to about 100 I.U. (or M.R.C.)/unit dosage.

Pharmaceutical compositions for use in accordance with the method of the invention may be prepared in accordance with standard galenical methods for the formulation of peptide preparations. Thus compositions for injection suitably comprise active ingredient, e.g. human or rat CGRP, in solution or suspension in an appropriate, e.g. aqueous, medium. Such compositions are appropriately put up, e.g. in ampoules for injection.

EXAMPLE 1

COMPOSITION CONTAINING HUMAN CGRP SUITABLE FOR INJECTION

| Ingredient | Quantity/ml |
|---|---|
| 1. Human CGRP | 20 µg |
| 2. NaCl | 7.5 mg |
| 3. HCl (IN) | added to pH 3.7 |
| 4. Distilled water: pharmaceutical grade | to an end volume of 1.0 ml. |

Components 1 to 4 are combined under protection of nitrogen gas (on a scale to produce a final volume of 2500 ml) in conventional manner, with 10 % excess component 1 being added initially to allow for later loss at filtration. The obtained solution is filtered, e.g. using a 0.2 µm filter and filled in 1.0 ml quantities into standard ampoules, suitable for use for injection.

Preparations comprising e.g. 0.1, 0.5, 10, 50 µg and 100 µg human CGRP are prepared entirely analogously by substitution of the desired quantity of component 1. If desired, the compositions may also incorporate a calcitonin in particular human or salmon calcitonin in an amount equivalent to e.g. 1.25 to 100 I.U. (or M.R.C.) for example 5, 10, 25, 50 or 100 I.U. (or M.R.C.) per ml end volume.

EXAMPLE 2

COMPOSITIONS CONTAINING CGRP AND A CALCITONIN SUITABLE FOR NASAL ADMINISTRATION

Compositions of the above type may be prepared entirely analogously to known formulations for the nasal administration of calcitonins, for example as described in DOS 3 335 086 and UK patent specification no. 2 127 689 A as well as in European patent application no. 115 627 A, but with incorporation of the desired amount of CGRP, e.g. rat or, especially, human CGRP. The following compositions are exemplary.

| (A) Ingredient | Quantity (per ml) |
|---|---|
| 1) Salmon calcitonin | 0.1375 mg |
| 10 % excess. | 0.01375 mg |
| | 0.15125 mg |
| 2) Human CGRP | 0.1 mg |
| 10 % excess. | 0.01 mg |
| | 0.11 mg |
| 3) NaCl | 7.5 mg |
| 4) Benzalkonium chloride | 0.1 mg |
| 5) HCl (1N) | added to pH 3.7 |
| 6) Distilled Water | to an end volume of 1.0 ml. |

Components 1 to 4 are combined under protection of nitrogen gas (on scale to produce a final volume of 2500 ml) in conventional manner, with 10 % of 1 and 2 being added to allow for loss at filtration. 5 is then added to bring the pH to 3.7 and distilled water added to a final end volume of 2500 ml. The obtained solution is filtered (e.g. using a 0.2 µm filter) to give a composition suitable for nasal application and for filling into a spray nasal dispenser with a solution volume of 2 ml. The composition comprises ca. 550 I.U. (or M.R.C.) calcitonin and ca. 100 µg CGRP/ml. The applicator delivers ca. 0.2 ml composition/actuation i.e. a quantity comprising ca. 55 I.U. (or M.R.C.) calcitonin and ca. 10 µg CGRP per actuation.

(B) The procedures of example 2A above are repeated using the same quantities of components 2 through 6 but employing the following quantities of component 1.

| Composition No. | Quantity of salmon calcitonin employed |
|---|---|
| B 1 | 0.01375 mg/ml |
| B 2 | 0.0275 mg/ml |
| B 3 | 0.06875 mg/ml |
| B 4 | 0.275 mg/ml |
| B 5 | 0.55 mg/ml |

The obtained compositions comprise ca. 100 µg CGRP/ml and ca. B 1 - 50; B 2 - 100; B 3 - 250; B 4 - 1,000; and B 5 - 2,000 I.U. (or M.R.C.) calcitonin/ml and are filled into a nasal spray dispensor delivering ca. 0.2 ml composition/actuation or 10 µg CGRP plus B 1 - 5; B 2 - 10; B 3 - 25; B 4 - 100; B 5 - 200 I.U. (or M.R.C.) calcitonin/actuation.

(C) The procedures of examples 2A and 2B 1 through 2B 5 above are repeated using the same quantities of components 1 and 3 through 6 but employing the following quantities of component 2.

| Composition No. | Quantity of human CGRP |
| --- | --- |
| C 1 | 0.0011 mg/ml |
| C 2 | 0.011 mg/ml |
| C 3 | 0.0275 mg/ml |
| C 4 | 0.275 mg/ml |
| C 5 | 1.1 mg/ml |

The obtained compositions comprise calcitonin in the concentrations previously indicated in example 2A and 2B and ca. C 1 - 0.1; C 2 - 1.0; C 3 - 2.5; C 4 - 25; and C 5 - 100 µg CGRP. They are filled into a nasal spray dispenser delivering ca. 0.2 ml composition/actuation or ca. C 1 - 0.01; C 2 - 0.1; C 3 - 0.25; C 4 - 2.5; and C 5 - 10 µg CGRP/actuation.

Compositions for nasal administration, e.g. as described above, may additionally comprise one or more surfactants as known in the art for the nasal administration of calcitonins.

Compositions, in particular nasal compositions for example as described above, comprising e.g. both a CGRP and a calcitonin, are novel and also form part of the present invention. Accordingly in a further aspect the present invention also provides:

i) A pharmaceutical composition comprising
  a) a peptide comprising the peptide sequence of a CGRP, or an analogue thereof,

and b) a calcitonin, together with one or more pharmaceutically acceptable diluents or carriers therefor.

Preferred components a) are rat and, in particular, human CGRP. Preferred components b) are human and salmon calcitonin.

Preferred compositions in accordance with the invention are compositions for nasal administration, e.g. compositions as defined which are suitable for, adapted for, or put up in a form suitable for nasal administration, for example suitable for administration in the form of a nasal spray or nasal drops, or put up in a nasal spray or drop applicator device.

Suitable relative quantities/concentrations of components a) and b) in the compositions of the invention are as hereinbefore described in relation to unit dosage formulation or as described in the above examples.

CLAIMS:

1. A method for the regulation of calcium balance, e.g. for the treatment of disease associated with or having an aetiology comprising body calcium imbalance, in a subject in need of such treatment, which method comprises administering to said subject an effective amount of a peptide comprising the peptide sequence of a CGRP or of an analogue thereof.

2. The use of a peptide comprising the peptide sequence of a CGRP or of an analogue thereof, for the regulation of calcium, e.g. for the treatment of disease associated with or having an aetiology comprising body calcium imbalance.

3. The use of a peptide comprising the peptide sequence of a CGRP or of an analogue thereof, for the manufacture of a composition for use in the regulation of calcium, e.g. for the treatment of disease associated with or having an aetiology comprising body calcium imbalance.

4. A method or use according to claim 1 or 2 for: or use according to claim 3 for the manufacture of a composition for use in: the treatment of hypercalaemia, Paget's disease, osteoporosis, bone pain associated with osteoporosis or osteolysis, or pain consequential to metastatic bone deposits.

5. A method or use according to any one of claims 1 to 4 wherein the peptide is rat CGRP.

6. A method or use according to any one of claims 1 to 4 wherein the peptide is human CGRP.

7. A method or use according to any one of claims 1 to 6 wherein the peptide is administered conjointly or concomitantly with a calcitonin.

8. A method or use according to claim 7 wherein the calcitonin is salmon or human calcitonin.

9. A pharmaceutical composition comprising
   a) a peptide comprising the peptide sequence of a CGRP, or an analogue thereof,
   and b) a calcitonin,
   together with one or more pharmaceutically acceptable diluents or carriers therefor.

10. A composition according to claim 9 for nasal administration.

FIG. 1

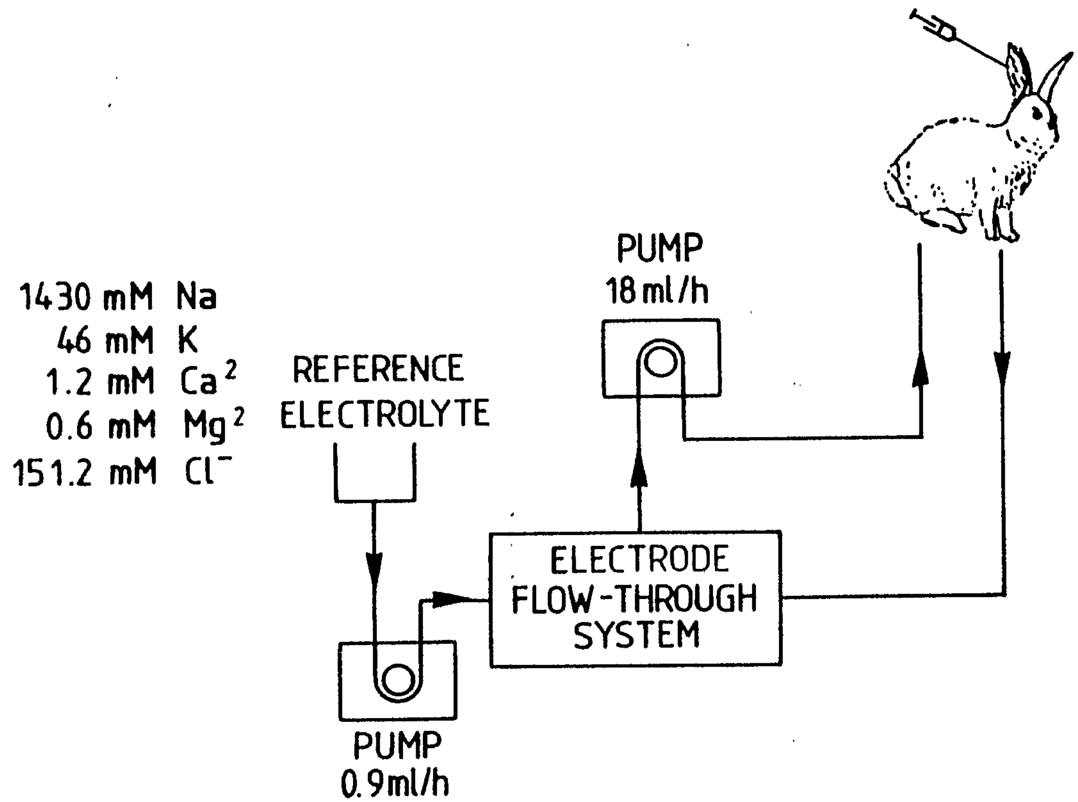
1430 mM Na
46 mM K
1.2 mM Ca 2
0.6 mM Mg 2
151.2 mM Cl⁻
REFERENCE ELECTROLYTE
PUMP 0.9ml/h
ELECTRODE FLOW-THROUGH SYSTEM
PUMP 18 ml/h

# FIG. 2

: hypocalcemia in Rabbit
– I.V. administration –

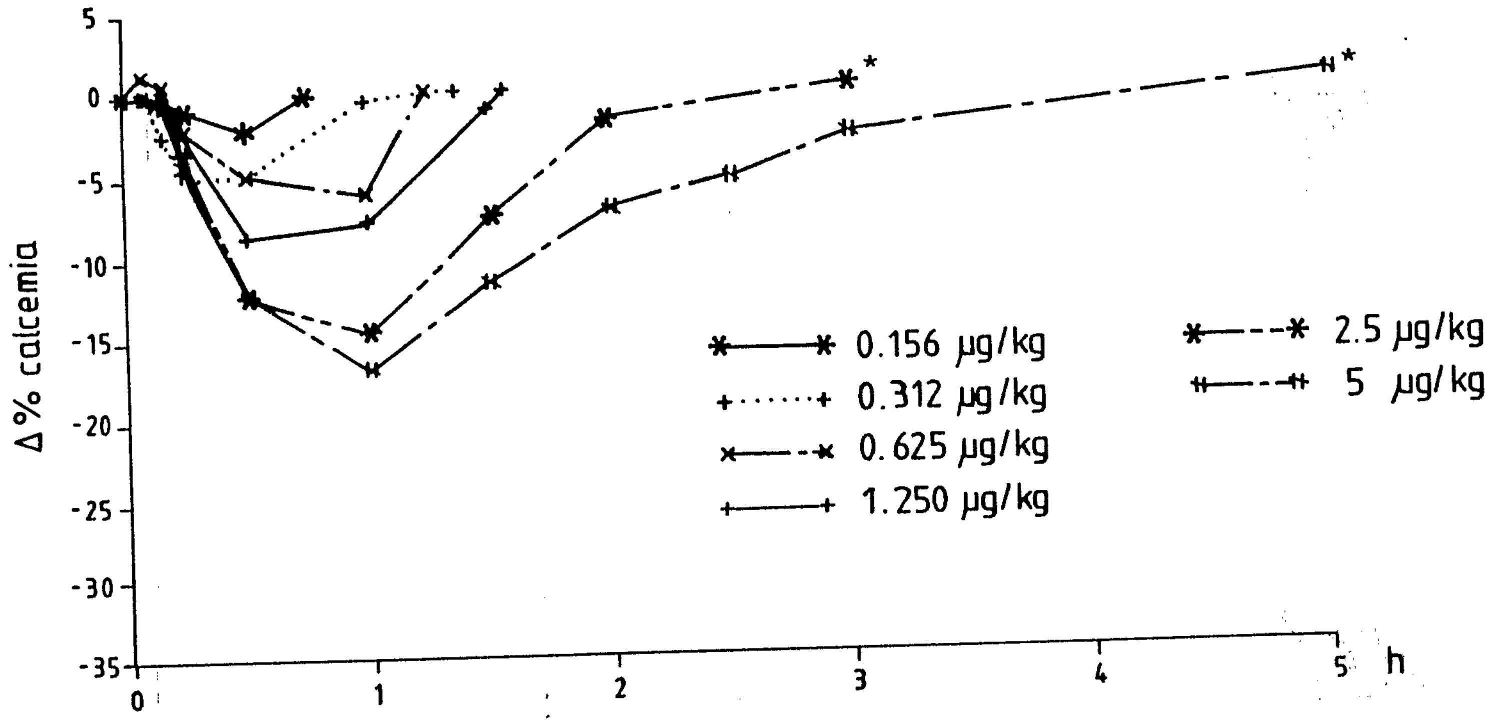


*extrapolated value

FIG. 3

2.5 μg/kg : Rabbit (n = 4)
- I.V. administration -

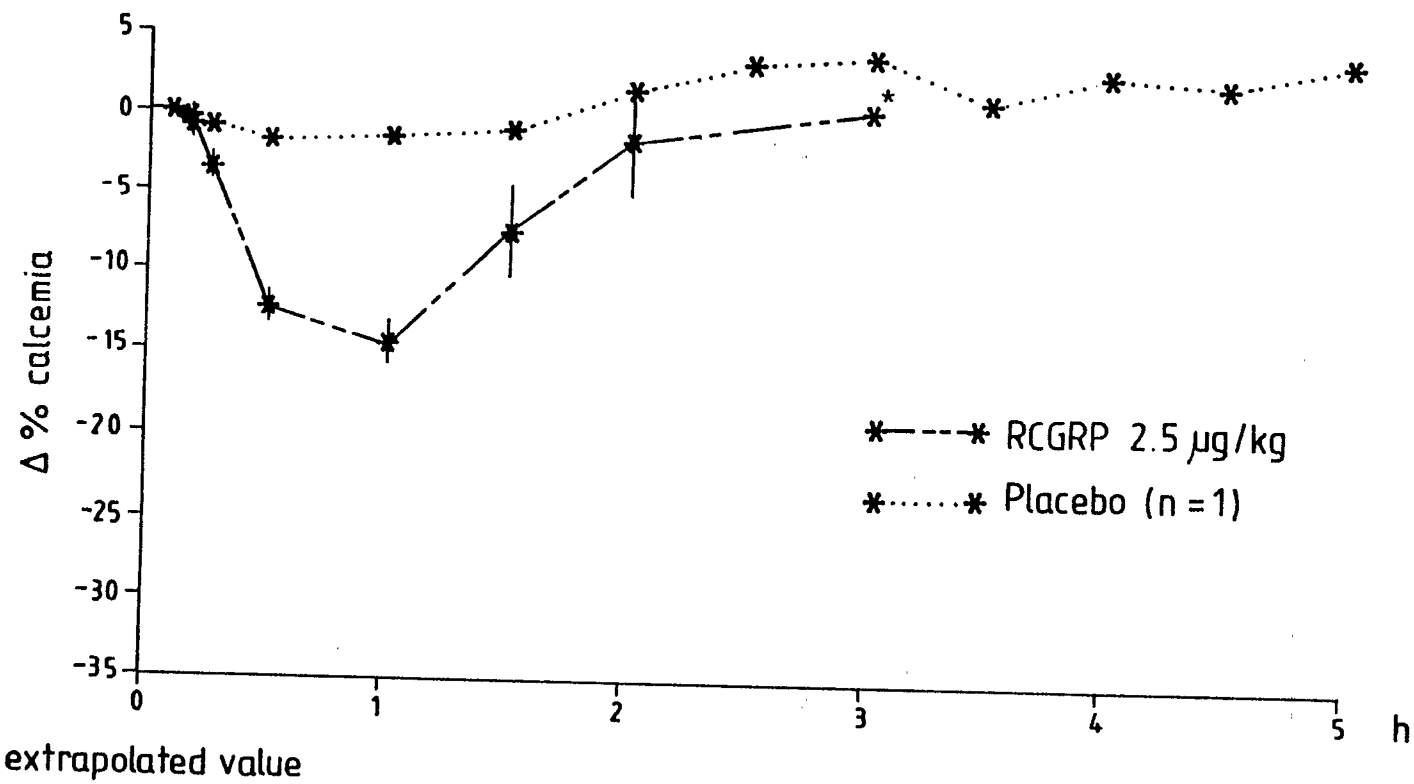


* extrapolated value

# FIG. 4

5 μg/kg : Rabbit (n = 4)
- I.V. administration -

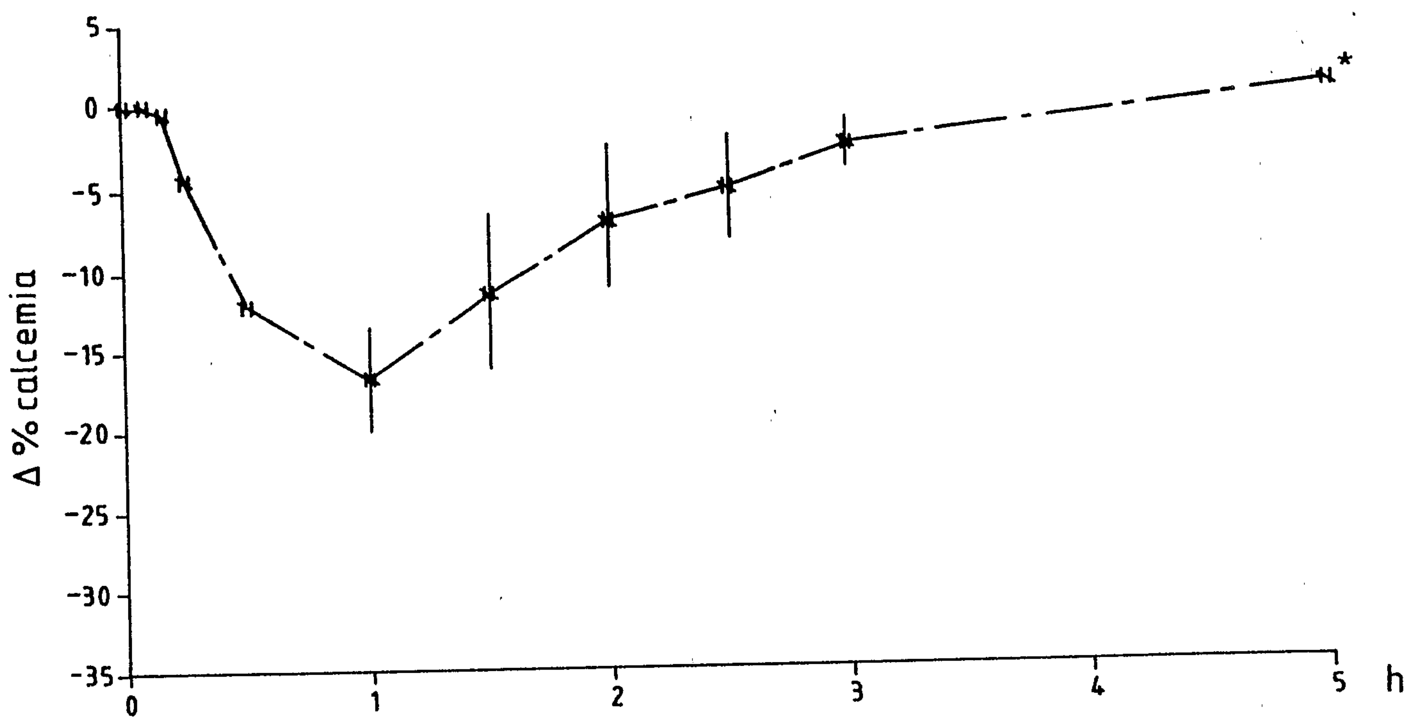


* extrapolated value

0156772

5/6

# FIG. 5

10 μg/kg Rabbit
- I V. administration -

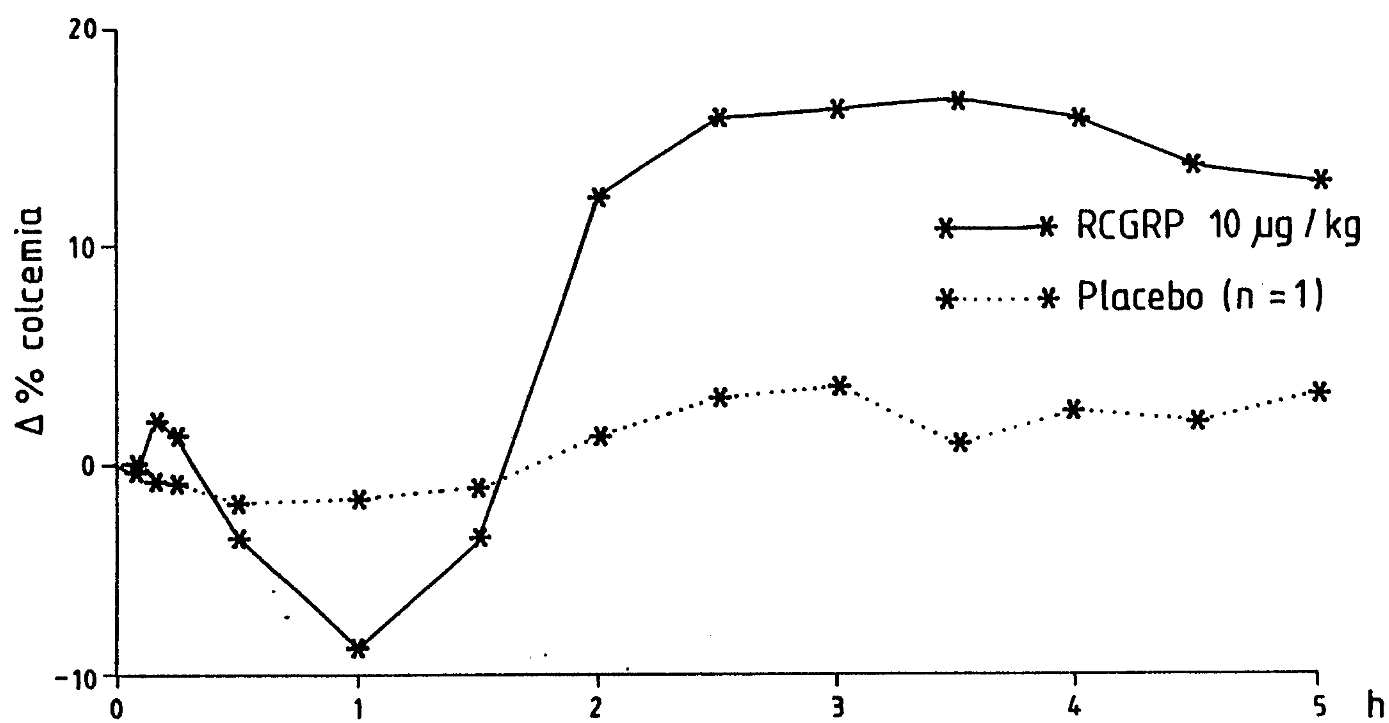

FIG. 6

Human CGRP

Variations of free Calcium in the young rabbit
after I.V. administration of 2.5 µg/kg. (n = 6)